# EUROPEAN PATENT SPECIFICATION

(11) **EP 1 460 999 B1**
(45) Date of publication and mention of the grant of the patent: **25.03.2009**
(21) Application number: 02804079.8
(22) Date of filing: 27.11.2002
(51) Int. Cl.: A61K 6/00, C07D 311/62, A61K 31/35, A61P 9/00

(54) **METHODS OF MAKING AND USING THEAFLAVIN, THEAFLAVIN-3-GALLATE, THEAFLAVIN-3'-GALLATE AND THEAFLAVIN 3,3'-DIGALLATE AND MIXTURES THEREOF**
VERFAHREN ZUR HERSTELLUNG UND VERWENDUNG VON THEAFLAVIN, THEAFLAVIN-3-GALLAT, THEAFLAVIN-3'-GALLAT UND THEAFLAVIN 3,3'-DIGALLAT UND IHRER GEMISCHE
PROCEDES DE FABRICATION ET D'UTILISATION DE THEAFLAVINE, DE THEAFLAVINE-3-GALLATE, DE THEAFLAVINE-3'-GALLAGE ET DE THEAFLAVINE 3,3'-DIGALLATE ET DES MELANGES DE CEUX-CI

(30) Priority: 28.11.2001 US 333515 P; 26.04.2002 CN 02111512; 24.09.2002 US 413576 P
(43) Date of publication of application: 29.09.2004
(73) Proprietor: Nashai Biotech, LLC, Brentwood, TN 37027 (US)
(72) Inventor: ZHAO, Jian, Irvine, CA 92614 (US); ZHOU, Rui, 201160 Shanghai (CN); CHEN, Hu Room 203, No 5 Lane, 200237 Shanghai (CN)
(74) Representative: Brasnett, Adrian Hugh
(86) International application number: PCT/US2002/038177
(87) International publication number: WO 2003/045328

(56) References cited:
- WO-A-01/93886
- DE-A1- 19 627 344
- US-A- 4 840 966
- US-A- 5 318 986
- US-A- 5 879 730
- A.FINGER: "in vitro studies on the effect of polyphenol oxidase a. peroxidase on the formation of polyphenolic black tea constituents." J. SCI. FOOD AGRIC., vol. 66, no. 3, 1994, pages 293-305, XP002312128 GB
- PATENT ABSTRACTS OF JAPAN vol. 1999, no. 05, 31 May 1999 (1999-05-31) & JP 11 032725 A (MITSUI NORIN KK), 9 February 1999 (1999-02-09)
- PATENT ABSTRACTS OF JAPAN vol. 0186, no. 51 (C-1285), 9 December 1994 (1994-12-09) & JP 6 256180 A (TAIYO KAGAKU CO LTD), 13 September 1994 (1994-09-13)
- CHEMICAL ABSTRACTS, vol. 120, no. 63, 1994, Columbus, Ohio, US; abstract no.: 144138v, page 691 column 1 XP002312132 & CN 1 074 618 A (HONGYAN FAMOUS TEA) 28 July 1993 (1993-07-28)
- CHEMICAL ABSTRACTS, vol. 127, no. 26, 1997, Columbus, Ohio, US; abstract no.: 355154e, MENG,MEI: "influence of theaflavin on blood lipid" page 25 column 2 XP002312133 & GUANGDONG YIXUE, vol. 18, no. 6, 1997, page 421, CHINA
- CHEMICAL ABSTRACTS, vol. 127, no. 14, 1997, Columbus, Ohio, US; abstract no.: 190040z, ISHIKAWA,T.: "effect of tea flavonoid supplementation" page 577 XP002312134 & AMERICAN JOURNAL OF CLINICAL NUTRITION, vol. 66, no. 2, 1997, pages 261-266, USA
- JEE Y. CHUNG: "mechanisms of inhibition of the ras-map kinase" FASEB JOURNAL, vol. 15, no. 11, September 2001 (2001-09), pages 2022-2024, XP002312129 US
- HASHIMOTO,F.: "evaluation of tea polyphenols as anti-hiv agents." BIOORGANIC A. MEDICINAL CHEMISTRY LETTERS, vol. 6, no. 6, 1996, pages 695-700, XP004135008 GB
- L.K. LEUNG,Z.-Y. CHEN: "theaflavin in black tea" JOURNAL OF NUTRITION, vol. 131, no. 9, 2001, pages 2248-2251, XP002312130 US
- H. YOSHIDA ET AL.: "inhibitory effect of tea flavonoids on the ability of cells to oxidize ldl" BIOCHEMICAL PHARMACOLOGY, vol. 58, no. 11, 1999, pages 1695-1703, XP002312131 GB

## Description

### Field Of The Invention

The present invention discloses methods of making a mixture of theaflavin, theaflavin-3-gallate, theaflavin-3'-gallate and theaflavin-3,3'-digallate, pharmaceutical compositions of the above mixture of theaflavins, diet supplement compositions of the above mixture of theaflavins and methods for using the above mixtures of theafiavin and pharmaceutical compositions thereof to treat or prevent various diseases. The present invention also discloses methods of making theaflavin, theaflavin-3-gallate, theaflavin-3'-gallate and theaflavin 3,3' -digallate, each as a separate compound.

### Background Of The Invention

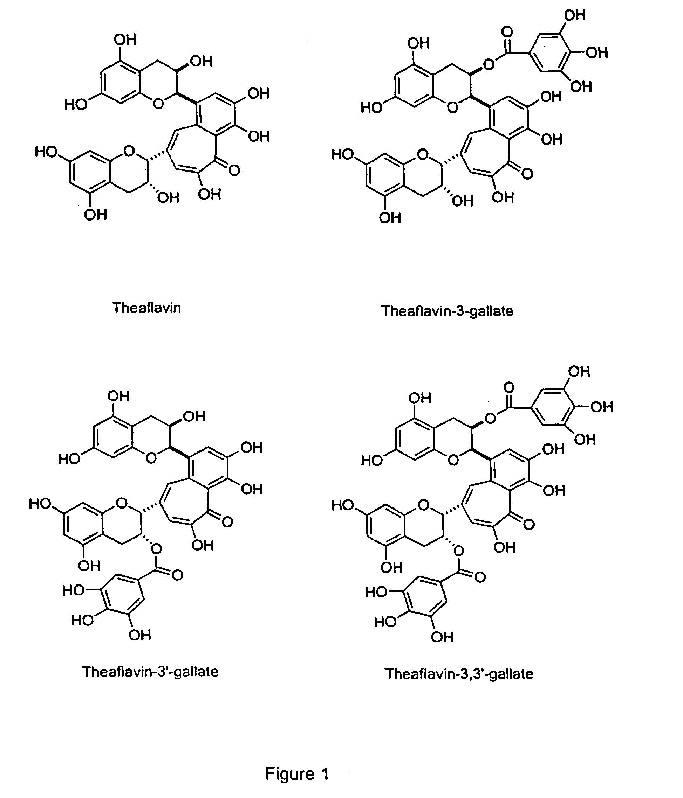

Tea, which is the most widely consumed beverage in the world other than water, is produced from the leaves of *Camellia Sinensis* and contain significant amounts of flavanoid compounds. Theaflavins, which comprise a mixture of theaflavin, theaflavin-3-gallate, theaflavin-3'-gallate and theaflavin-3,3'-digallate, as depicted in Figure 1, are typically formed via polymerization of green tea polyphenols (*i.e*., flavanoids) during fermentation of green tea to black tea. Typically, the concentration of theaflavins in black tea is between about 0.4% to about 1.8% by weight with the concentration of theaflavins in green tea usually being far less.

Theaflavins are responsible for the characteristic color (*i.e*., brightness) and flavor (*i.e*., briskness) ofblack tea. Flavanoids are effective anti-oxidants (Leung et al., J Nutr 2001, 131(9):2248-51, Sarkar et al., Biochem Biophys Res Commun 2001, 284(1):173-8; Yoshino et al., Biol. Pharm Bull 1994,17(1) 146-149) and may be efficacious against various diseases such as cancer, cardiovascular and cerebrovascular diseases, diabetes, *etc.* Further, flavanoids may possess significant anti-inflammatory antimicrobial and antiviral activity.

However, a significant problem in using theaflavins to treat or prevent various diseases is isolating theaflavins, either as a mixture or as individual compounds, in sufficient quantities, from naturally occurring sources using an economical procedure. Thus, what is needed are methods for isolating theaflavins, either as a mixture or as individual compounds, in sufficient quantities in a cost effective manner so that theaflavins, either as a mixture or as individual compounds, may be used to treat or prevent various diseases.

Finger, A. (J. Sci. Food Agric, 1994, 66:293-345) describes in vitro studies on the effect of polyphenol oxidase and peroxidase on the formation of polyphenolic black tea constituents. JP-A-06-256180 describes an intestinal environment improvement composition containing tannins and water-soluble dietary fibre. Leung et al., (journal of Nutrition, 2001, 131:2248-2251) compare the antioxidant activities of theaflavins with that of catechins. DE-A1-19627344 refers to a nutritional supplement and/or pharmaceutical preparation containing a minimum of one of a number of polyphenol compounds.

### Summary Of The Invention

The present invention satisfies these and other needs by providing methods of making a mixture of theaflavin, theaflavin-3-gallate, theaflavin-3'-gallate and theaflavin 3,3'-digallate and methods of making theaflavin, theaflavin-3-gallate, theaflavin-3'-gallate and theaflavin 3,3'-digallate, each as a separate compound and methods of using the mixture of theaflavins to treat or prevent various diseases. Importantly, the methods above are efficiently carried out on large scale at minimal cost.

In one aspect, the current invention provides a method of making a mixture of theaflavin, theaflavin-3-gallate, theaflavin-3'-gallate and theaflavins 3,3'-digallate. Green tea leaves are contacted with an aqueous buffer to form a reaction mixture. The reaction mixture is then contacted with oxygen to begin fermentation and is fermented for a time sufficient to form the mixture of theaflavin, theaflavin-3-gallate, theaflavin-3'gallate and theaflavin 3,3'-digallate. Fermentation is then terminated and the reaction mixture is separated from the mixture of theaflavin, theaflavin-3-gallate, theaflavin-3'-gallate and theaflavin 3,3' digallate.

In another aspect, described herein are pharmaceutical compositions comprising the mixture of theaflavin, theaflavin-3-gallate, theaflavin-3'-gallate and theaflavin-3,3'-digallate or a pharmaceutically available salt, hydrate or solvate thereof, The pharmaceutical compositions generally comprise the mixture of theaflavin, theaflavin-3-gallate, theaflavin-3'-gallate and theaflavin-3,3'-digallate or a pharmaceutically available salt, hydrate or solvate thereof and a suitable excipient, carrier or diluent. The composition may be formulated for veterinary uses or for use in humans.

Thus, the invention provides:
- a pharmaceutical composition comprising:
   (a) as the pharmaceutically active agent, an isolated mixture of theaflavin, theaflavin-3-gallate, theaflavin-3'-gallate, and theaflavin-3,3'-digallate; and
   (b) one or more physiologically acceptable excipients, carriers, or diluents;
   wherein the composition is for administration of the mixture (a) at a dose in the range of 0.001 mg to 200mg/kg body weight.
- a pharmaceutical composition comprising:
   (a) as the pharmaceutically active agent, a mixture of theaflavin, theaflavin-3-gallate, theaflavin-3'-gallate, and theaflavin-3,3' -digallate; and
   (b) one or more physiologically acceptable excipients, carriers, or diluents;
   wherein the theaflavin mixture is present at a level of between 5% and 50% (w/w) in the composition.
- a pharmaceutical composition consisting of:
   (a) as the pharmaceutically active agent, a mixture of theaflavin, theaflavin-3-gallate, theaflavin-3'-gallate, and theaflavin-3,3'-digallate; and
   (b) one or more physiologically acceptable excipients, carriers or diluents;
   wherein the composition is for administration of the mixture (a) at a dose in the range of 0.00 1mg to 200mg/kg body weight.

In still another aspect, described herein are diet supplement compositions comprising the mixture of theaflavin, theaflavin-3-gallate, theaflavin-3'-gallate and theaflavin-3,3-digallate. The diet supplement compositions generally comprise the mixture of theaflavin, theaflavin-3-gallate, theaflavin-3'-gallate and theaflavin-3,3'-digallate or a pharmaceutically available salt, hydrate or solvate thereof and a suitable diet supplement vehicle. Thus, the invention provides a dietary supplement composition consisting of:
a mixture of theaflavin, theaflavin-3-gallate, theaflavin-3'-gallate, and theaflavin-3,3'-digallate;
and a suitable diet supplement vehicle;
wherein the composition is for administration of the mixture (a) at a dose in the range of 0.001mg to 200mg/kg body weight.

Described herein are methods for treating or preventing various diseases, which include but are not limited to, hyperlipidemia, coronary heart disease, apoplexy, atherosclerotic cardiovascular disease, diabetes, leucopenia, cerebral infarction and dementia or physical disorder. The methods may comprise administering to a patient in need of such treatment or prevention a therapeutically effective amount of the mixture of theaflavin, theaflavin-3-gallate, theaflavin-3'-gallate and theaflavin-3-3'-digallate or a pharmaceutically available salt, hydrate or solvate thereof, Alternatively, the methods may comprise administering to a patient in need of such treatment or prevention a therapeutically effective amount of the above pharmaceutical composition of the mixture of theaflavins. In one aspect, the invention provides a mixture of theaflavin, theaflavin-3-gallate, theaflavin-3'-gallate, and theaflavin-3,3'-digallate for use in a method of treating or preventing hyperlipidemia and a mixture of theaflavin, theaflavin-3-gallate, theaflavin-3'-gallate, and theaflavin-3,3'-digallate for use in a method of decreasing low density lipoprotein-cholesterol (LDL-c) levels without significantly changing high density lipoprotein-cholesterol (HDL-c) levels. The invention further provides use of a mixture of theaflavin, theaflavin-3-gallate, theaflavin-3'-gallate, and theaflavin-3,3'-digallate in the manufacture of a medicament for treating or preventing hyperlipidemia and use of a mixture of theaflavin, theaflavin-3-gallate, theaflavin-3'-gallate, and theaflavin-3,3'-digallate in the manufacture of a medicament for decreasing LDL-c levels without significantly changing HDL-c levels.

In still another aspect, the current invention provides a method of making theaflavin, theaflavin-3-gallate, theaflavin-3'-gallate or theaflavin-3,3'-digallate, each as a separate compound. Tea polyphenols are contacted with an aqueous buffer and polyphenol oxidase to form a reaction mixture. The reaction mixture is then contacted with oxygen to begin fermentation and is fermented for a time sufficient to form a mixture of theaflavin, theaflavin-3-gallate, theaflavin-3'-gallate and theaflavin-3,3'-digallate. Fermentation is then terminated and the reaction mixture is separated to provide theaflavin, theaflavin-3-gallate, theaflavin-3'-gallate and theaflavin-3,3'-digallate, each as a separate compound

### Brief Description Of The Drawing

Figure 1 illustrates the chemical structure of theaflavin, theaflavin-3-gallate, theaflavin-3'-gallate and theaflavin-3,3'-digallate; and

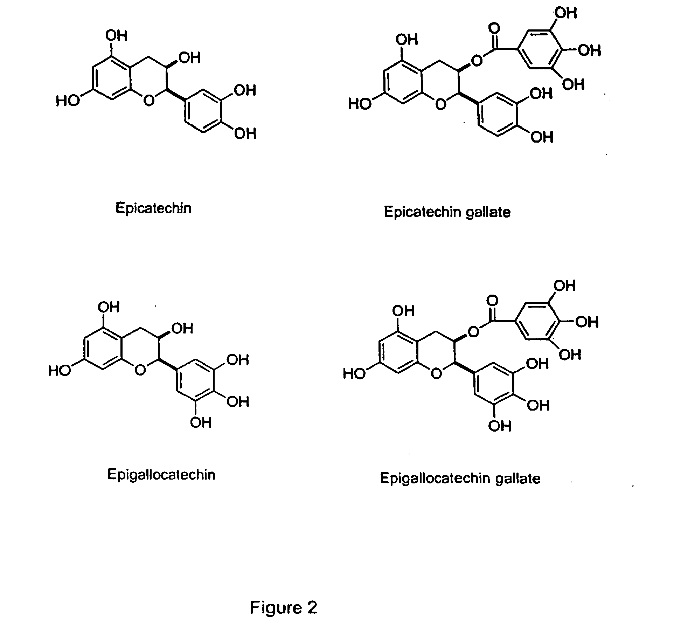

Figure 2 illustrates the chemical structure of epicatechin, epicatechin gallate, epigallocatechin and epigallocatechin.

### Detailed Description Of The Invention

### Definitions

"Buffer" refers to a substance which binds reversibly with hydrogen ion and consequently resists change in pH upon addition of acid or base. Examples of buffers include, but are not limited to, proteins, bicarbonates, carbonates, carboxylates (*e.g*., acetate, propionate, *etc*.), sulfonates, phosphates (*e.g*., sodium phosphate), citrates, borates, amino acids *(e.g,* glycine, alanine, *etc*.) and amines (*e.g*., methylamine, ethylamine, Tris, *etc.).*

"Compounds of the invention" refers to any mixture of the theaflavins as well as theaflavin, theaflavin-3-gallate, theaflavin-3'-gallate and theaflavin 3,3'-digallate individually. The compounds of the invention may be identified either by their chemical structure and/or chemical name. When the chemical structure and chemical name conflict, the chemical structure is determinative of the identity of the compound. The compounds of the invention may also exist in several tautomeric forms including the enol form, the keto form and mixtures thereof. Accordingly, the chemical structures depicted herein encompass all possible tautomeric forms of the illustrated compounds. The compounds of the invention may also include isotopically labeled compounds where one or more atoms have an atomic mass different from the atomic mass conventionally found in nature. Examples of isotopes that may be incorporated into the compounds of the invention include, but are not limited to, ²H ³H, ¹³C, ¹⁴C, ¹⁸O and ¹⁷O. Compounds of the invention may exist in unsolvated forms as well as solvated forms, including hydrated forms. In general, hydrated and solvated forms are within the scope of the present invention. Certain compounds of the present invention may exist in multiple crystalline or amorphous forms- In general, all physical forms are equivalent for the uses contemplated herein.

"Green tea leaves" refers to leaves of the evergreen species *Camellia Sinensis* which have been withered, heated (*e.g*., pan fried or steamed) to prevent the oxidation (*i.e*., fermentation) process and then naturally dried.

"Pharmaceutically acceptable salt" refers to a salt of a compound of the invention, which is pharmaceutically acceptable and possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1;2-ethatee-disulfooic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid and the like; or (2) salts formed when an acidic proton present in the parent compound is replaced by an ammonium ion, a metal ion, *e.g*., an alkali metal ion *(e.g.,* sodium or potassium), an alkaline earth ion (*e.g*., calcium or magnesium), or an aluminum ion; or coordinates with an organic base such as ethanolamine, diethanolamine, triethanolamine, N-methylglucamine, morpholine, piperidine, dimethylamine, diethylamine and the like. Also included are salts of amino acids such as arginates and the like, and salts of organic acids like glucurmic or galactunoric acids and the like *(see, e.g.,* Berge et al., 1977, J. Pharm. Sci. 66:1-19).

"Pharmaceutical acceptable vehicle" refers to a diluent, adjuvant, excipient or carrier with which a compound of the invention is administered.

"Patient" includes humans and other mammals.

"Preventing" or "prevention" refers to a reduction in risk of acquiring a disease or disorder (*i.e*., causing at least one of the clinical symptoms of the disease not to develop in a patient that may be exposed to or predisposed to the disease but does not yet experience or display symptoms of the disease).

"Theaflavins" refers to those compounds that are formed by the oxidative condensation of a catechin quinone (*e.g*., epicatechin) and a gallocatechin quinone *(e.g.,* epigallocatchin), a reaction which may be catalyzed by the enzyme polyphenol oxidase. Theaflavins include theaflavin, theaflavin-3-gallate, theaflavin-3'-gallate and theaflavin 3,3'-digallate. Theaflavins are soluble in hot water, ethyl acetate, butanol and methanol, *etc.* and are typically sensitive to oxidation in basic solution.

"Tea polyphenols" refers to a mixture of polyphenols extracted from green tea leaves with catechins comprising about 90% of the mixture. Tea polyphenols are available from commercial sources (*e.g*., Wuzhou International Co., LTD., Ningbo Free Trade Zone, Ningbo, China). Catechins include gallocatechin, epicatechin ("EC"), epigallocatechin ("EGC") epicatechin gallate ("EGC"), epigallocatechin gallate ("EGCG"). Typically EGCG comprises about 50% to about 60% of the catechin content.

"Treating" or "treatment" of any disease or disorder refers, in one embodiment, to ameliorating the disease or disorder (*i.e*., arresting or reducing the development of the disease or at least one of the clinical symptoms thereof). In another embodiment "treating" or "treatment" refers to ameliorating at least one physical parameter, which may not be discernible by the patient. In yet another embodiment, "treating" or "treatment" refers to inhibiting the disease or disorder, either physically, (*e.g*., stabilization of a discernible symptom), physiologically, (*e.g*., stabilization of a physical parameter), or both. In yet another embodiment, "treating" or "treatment" refers to delaying the onset of the disease or disorder. "Therapeutically effective amount" means the amount of a compound that, when administered to a patient for treating or preventing a disease, is sufficient to effect such treatment or prevention of the disease. The "therapeutically effective amount" will vary depending on the compound, the disease and its severity and the age, weight, *etc.,* of the patient to be treated.

### Method of Making Theaflavin Mixtures and Individual Theaflavins

In one aspect, the current invention provides a method of making a mixture of theaflavin, theaflavin-3-gallate, theaflavin-3'-gallate and theaflavin-3,3'-digallate. First, green tea leaves, are contacted with an aqueous buffer to form a reaction mixture. Green tea leaves may be purchased from commercial sources (*e.g*., Far Tea Leaves, Berkeley, CA; SOTA Instant Japanese Green Tea, Reno, Nevada; FML Tea Trading Co., Ltd., Xiamen, China) and may be used as provided by the supplier or maybe shredded prior to contacting with buffer.

Preferably, the aqueous buffer has a pH of between about 5.0 and about 7.0 (more preferably, between about 6.3 and about 6.5). The molar amount of buffer should be enough to buffer the aqueous solution and may be readily approximated by the skilled artisan. In one embodiment, the buffer is between about 0.1 M and about 0.3 M. The buffer may be selected from any buffering agent which can be adjusted to the desired pH of the reaction mixture. Methods for selecting an appropriate buffering agent are well know to the skilled artisan. Preferred buffers include phosphate. In one embodiment, the buffering agent is 0.15 M phosphate at pH 6.4. In another embodiment, the ratio of green tea leaves to aqueous buffer is between about 1:0.5 and about 1:20 on a kilogram to liter basis.

In some cases, the green tea leaves may contain enough residual polyphenol oxidase to convert catechins to theaflavins. In other situations, polyphenol oxidase (Worthington Biochemical Corporation, Lakewood, NJ) may be added to the reaction mixture to assist in formation of theaflavins from catechins. Typically, the ratio of polyphenol oxidase to green tea leaves may vary between about 0.1:1 and about 2:1 (w/w).

The reaction mixture is then contacted with oxygen to initiate fermentation. Typically the reaction is agitated and preferably, is stirred. The amount of oxygen in the reaction mixture may vary between about 20% and about 25% (w/v). The appropriate oxygen flow rate through the reaction mixture will vary with the reaction volume and may be readily determined by the skilled artisan. In one embodiment, the amount of oxygen in the reaction mixture is about 22% (w/v). The temperature of the reaction mixture may vary between about 15 °C and about 80 °C. In one embodiment, the temperature is about 30°C. In another embodiment, the amount of oxygen in the reaction mixture is about 22% (w/v), the flow rate of oxygen through the reaction mixture is about 20 L/sec, the volume of the reaction mixture is between about 1000 L and about 1200 L, the temperature is about 30 °C and the reaction mixture is stirred.

The reaction mixture is then fermented to form the mixture of theaflavin, theaflavin-3-gallate, theaflavin-3'-gallate and theaflavin-3,3'-digallate. Typically, the reaction mixture is fermented between about 0.5 hours and about 1.5 hours. In one embodiment, the reaction mixture is fermented for about 1 hour. It should be noted that the temperature and oxygen concentration of the reaction mixture remain roughly constant during fermentation. Preferably, the reaction mixture is agitated during fermentation, more preferably, the reaction mixture is stirred during fermentation.

Finally, the reaction mixture is separated from the mixture of theaflavin, theaflavin-3-gallate, theaflavin-3'-gallate and theaflavin-3,3'-digallate. Preferably, any solids in the reaction mixture are removed. The reaction mixture may be filtered to remove solids. More preferably, the reaction mixture is centrifuged to remove solids. Even more preferably, the reaction mixture is both centrifuged and filtered to remove solids.

The filtered reaction mixture is then contacted with organic solvent, which terminates fermentation. The organic solvent should dissolve appreciable quantities of theaflavins *(i.e.,* theaflavins should be more than sparingly soluble in the selected solvent) and be at least partially immiscible with aqueous solutions (*i.e*., the aqueous phase and the organic phase should form two phases after mixing). The organic solvent may be selected without undue experimentation by those of skill in the art given the above criteria. In a preferred embodiment, the organic solvent is ethyl acetate. It should be noted that mixtures of organic solvents can be used in the extractive method, provided the solvent mixture satisfies the above criteria.

Calculating the volume of organic solvent needed for extraction of theaflavins from aqueous solution are well within the capability of the skilled artisan. Preferably, the volume of organic solvent used to extract theaflavins from aqueous solution is between about 1/3 of the volume of the aqueous solution and about three times the volume of the aqueous solution. More preferably, the volume of organic solvent used to extract theaflavins from aqueous solution is about equal to the volume of the aqueous solution.

The aqueous solution may be contacted with as many portions of organic solvent necessary to extract substantially all of the theaflavins into the organic phase. The number of portions of organic solvent necessary to completely extract theaflavins from aqueous solution may be readily determined by one of skill in the art without undue experimentation.

Generally, contacting organic solvent and aqueous solution in a separatory funnel is sufficient to extract theaflavins into organic solvent from aqueous solution. However, in some situations, the organic solvent and aqueous solution may be contacted by other methods well known to the skilled artisan such as magnetic stirring, mechanical stirring, sonication, *etc*.

The organic solvent containing the theaflavins is then contacted with dilute aqueous base. Preferably, the base is dilute hydroxide, carbonate or bicarbonate. In one embodiment, the dilute aqueous base is 5% NaHCO₃ which is contacted with organic solvent in a separatory funnel. While not wishing to bound by theory, contacting the organic solvent with dilute base presumably removes base soluble impurities from the mixture of theaflavins. The solvent is next separated from the dilute aqueous base solution, preferably, by using a separatory funnel.

In one embodiment, the concentration of the mixture of theaflavins in the reaction mixture after extractive workup comprises between about 5% and about 90% of the total mass of non-volatile material. In another embodiment, the concentration of the mixture of theaflavins in the reaction mixture after extractive workup comprises between about 10% and about 80% of the total mass of non-volatile material. In another embodiment, the concentration of the mixture of theaflavins in the reaction mixture after extractive workup comprises between about 20% and about 70% of the total mass of non-volatile material.

The separated organic solvent is then contacted with a chromatographic media to provide the mixture of theaflavin, theaflavin-3-gallate, theaflavin-3'-gallate and theaflavin-3,3'-digallate. Preferably, the organic solution is concentrated before contacting with the chromatographic media. The chromatographic media may be any chromatography support known to those of skill in the art. In one embodiment, the chromatographic media is silica gel. Preferably, the organic solvent containing the mixture of theaflavins is loaded onto chromatographic media, which has been packed in a column and eluted with a second organic solvent to provide the mixture of theaflavin, theaflavin-3-gallate, theaflavin-3'-gallate and theaflavin-3,3'-digallate. In one embodiment, the organic solvent containing the mixture of theaflavins is loaded onto a silica gel column and is eluted with 40% ethyl acetate in hexanes. In some situations, batch use of chromatographic media may be preferred. Selection of the type and amount of chromatographic media, choice of eluting solvent, length of column, *etc.* is within the ambit of those of skill in the art.

In one embodiment, the mixture of theaflavin, theaflavin-3-gallate, theaflavin-3'-gallate and theaflavin-3,3'-digallate is substantially pure *(i.e.,* free of other material) after elution from the chromatographic media. Preferably, the mixture of theaflavin, theaflavin-3-gallate, theaflavin-3'-gallate and theaflavin-3,3'-digallate is greater than 95% pure, more preferably, greater than 97% pure and most preferably, greater than 99% pure as determined by routine analytical methods well known to those of skill in the art.

The proportion of the individual theaflavins in the mixture of theaflavins will reflect the proportion of individual theaflavins in green tea leaves and will, accordingly, vary with source of green tea leaves. In one embodiment, the mixture of theaflavins is comprised of between about 40% and about 50% theaflavin, between about 15% and about 25% of theaflavin-3-gallate, between about 10% and about 14% theaflavin-3'-gallate and between about 15% and about 25% of theaflavin-3,3'-digallate. In another embodiment, the mixture of theaflavins is comprised of about 47% theaflavin, about 19% of theaflavin-3-gallate, about 14% theaflavin-3'-gallate and about 20% of theaflavin-3,3'-digallate.

In a second aspect, the current invention provides a method of making theaflavin, theaflavin-3-gallate, theaflavin-3'-gallate and theaflavin-3,3'-digallate, each as a separate compound. First, tea polyphenols, are contacted with an aqueous buffer to form a reaction mixture. Tea polyphenols may be purchased from commercial sources (Yuan Xiang Biopharm Company, Ltd., Shanghai, China) and may be used as provided by the supplier. Polyphenol oxidase (Worthington Biochemical Corporation, Lakewood, NJ) is added to the reaction mixture. Typically, the ratio of polyphenol oxidase to tea polyphenols may vary between about 0.5:1 and about 1:1 on a w/w basis. The remainder of the method may be performed as described above for forming the mixture of theaflavin, theaflavin-3-gallate, theaflavin-3'-gallate and theaflavin-3,3'-digallate from green tea leaves except for the final purification on a chromatographic media. Preferably, the mixture of theaflavins is divided into a number of different portions, which are then individually applied to columns of chromatographic media to provide theaflavin, theaflavin-3-gallate, theaflavin-3'-gallate and theaflavin-3,3'-digallate, each as a single compound rather than as a mixture of compounds. Selection of the parameters needed to resolve the mixture of theaflavins into theaflavin, theaflavin-3-gallate, theaflavin-3'-gallate and theaflavin-3,3'-digallate each as a single compound (*i.e*., number of portions, selection of type and amount of chromatographic media, choice of eluting solvent, length of column, *etc.)* is well within the ambit of those of skill in the art.

### Therapeutic Uses

The compounds of the invention may be used to treat a variety of diseases and disorders, which include but are not limited to, hyperlipidemia, coronary heart disease, apoplexy, arthesclerotic cardiovascular diseases, AIDS, diabetes, oxidated-low density lipoprotein level, von Willebrand's disease, leukopenia, cerebral infarction, dementia and physical disorder, fatty liver- Description of the symptoms of these diseases and disorders may be found, for example, in the Merck Manual, sixteenth edition.

A compound of the invention and/or a pharmaceutical composition thereof may be administered to a patient, preferably a human, suffering from hyperlipidemia.

Further, compounds of the invention and/or pharmaceutical compositions thereof may be admmistered to a patient, preferably a human, as a preventative measure against the above diseases or conditions. Thus, the compounds of the invention and/or pharmaceutical compositions thereof may be administered as a preventative measure to a patient having a predisposition for any of the above diseases or disorders. Accordingly, the compounds of the invention and/or pharmaceutical compositions thereof may be used for the treating or preventing one disease or disorder and concurrently treating or preventing another (*e.g*., preventing hyperlipidemia while treating a cerebral infarction).

The suitability of the compounds of the invention and/or pharmaceutical compositions thereof in treating or preventing the various diseases or disorders listed above may be determined by methods described in the art. Accordingly, it is well within the capability of those of skill in the art to assay and use the compounds of the invention and/or pharmaceutical compositions thereof to treat or prevent the above diseases or disorders.

### Therapeutic/Prophylactic Administration

The compounds of the invention and/or pharmaceutical compositions thereof may be advantageously used in human and veterinary medicine. As previously described in Section 4.3 above, compounds of the invention and/or pharmaceutical compositions thereof are useful for the treatment or prevention of various diseases or disorders listed above.

When used to treat or prevent the above diseases or disorders, compounds of the invention and/or pharmaceutical compositions thereof may be administered or applied singly, or in combination with other agents. The compounds of the invention and/or pharmaceutical compositions thereof may also be administered or applied singly, in combination with other pharmaceutically active agents including other compounds of the invention and/or pharmaceutical compositions thereof.

Described herein are methods of treatment and prophylaxis by administration to a patient of a therapeutically effective amount of a compound of the invention and/or pharmaceutical composition thereof The patient is preferably a mammal and most preferably, is a human.

The compounds of the invention and/or pharmaceutical compositions thereof may be administered orally, which results in the release of the compounds of the invention and/or pharmaceutical compositions thereof into the bloodstream. The compounds of the invention and/or pharmaceutical compositions thereof can be delivered via oral sustained release systems. In one embodiment, polymeric materials are used for oral sustained release delivery. Preferred polymers include sodium carboxymethylcellulose, hydroxypropylcellulose, hydroxypropylmethylcellulose and hydroxyethylcehulose (most preferred, hydroxypropyl methylcellulose). Other preferred cellulose ethers have been described (Alderman, Int. J. Pharm. Tech. & Prod. Mfr. 1984, 5(3) 1-9). Factors affecting drug release are well known to the skilled artisan and have been described in the art (Bamba et al., Int. J. Pharm. 1979, 2,307).

Enteric-coated preparations can be used for oral sustained release administration. Preferred coating materials include polymers with a pH-dependent solubility (*i.e*., ph-controlled release), polymers with a slow or pH-dependent rate of swelling, dissolution or erosion (*i.e*., time-controlled release), polymers that are degraded by enzymes (*i.e*., enzyme-controlled release) and polymer; that form firm layers that are destroyed by an increase in pressure (*i.e*., pressure-controlled release).

Osmotic delivery systems may be used for oral sustained release administration (Verma et al., Drug Dev. Ind. Pharm., 2000,26:695-708).

OROS^{™} osmotic devices may be used for oral sustained release delivery devices (Theeuwes *et al.,* United States Patent No. 3,845,770; Theeuwes *et al.,* United States Patent No. 3,916,899).

### Pharmaceutical Compositions

The pharmaceutical compositions described herein typically contain a therapeutically effective amount of a compound of the invention, preferably in purified form, together with a suitable amount of a pharmaceutically acceptable vehicle, so as to provide the form for proper administration to a patient. The compounds of the invention may be present at a level of between about 5%and about 50%(w/w), preferably, about 11% in the pharmaceutical composition. Total amount of the compound of the invention per dose may be between about 70 mg and about 210 mg.

When administered to a patient, the compounds of the invention and pharmaceutically acceptable vehicle are preferably sterile. Water, saline solutions and aqueous dextrose and glycerol solutions may be employed as liquid vehicles. Other suitable pharmaceutical vehicles include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present pharmaceutical compositions, if desired, can also contain minor amounts of wetting or emulsifying agents or pH buffering agents. In addition, auxiliary, stabilizing, thickening, lubricating and coloring agents may be used. A general discussion of the preparation of pharmaceutical compositions may be found in Remington, "The Science and Practice of Pharmacy," 19th Edition.

Pharmaceutical compositions comprising a compound of the invention may be manufactured by means of conventional mixing, dissolving, granulating, dragee-making, levigating, emulsifying, encapsulating, entrapping or lyophilizing processes. Pharmaceutical compositions may be formulated in conventional manner using one or more physiologically acceptable carrier, diluents, excipients or auxiliaries, which facilitate processing of compounds of the invention into preparations which can be used pharmaceutically.

The pharmaceutical compositions described herein can take the form of solutions, suspensions, emulsion, tablets, pills, pellets, capsules, capsules containing liquids, powders, sustained-release formulations, emulsions or any other form suitable for oral use. In one embodiment, the pharmaceutically acceptable vehicle is a capsule (see *e.g*., Grosswald *et al.,* United States Patent No. 5,698,155). Other examples of suitable pharmaceutical vehicles have been described in the art (see Remington, "The Science and Practice of Pharmacy," 19th Edition, 1995). Orally administered pharmaceutical compositions may contain one or more optional agents, for example, sweetening agents such as fructose, aspartame or saccharin; flavoring agents such as peppermint, oil of wintergreen, or cherry coloring agents and preserving agents, to provide a pharmaceutically palatable preparation. Moreover, when in tablet or pill form, the pharmaceutical compositions may be coated to delay disintegration and absorption in the gastrointestinal tract, thereby providing a sustained action over an extended period of time. Selectively permeable membranes surrounding an osmotically active driving compound are also suitable for orally administered compounds of the invention. In these later platform, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time delay material such as glycerol monostearate or glycerol stearate may also be used. Oral compositions can include standard vehicles such as mamitol, lactose, starch, magnesium stearate, sodium saccharine, cellulose, magnesium carbonate, *etc.* Such vehicles are preferably of pharmaceutical grade.

For oral liquid preparations such as, for example, suspensions, elixirs and solutions, suitable carriers, excipients or diluents include water, saline, alkyleneglycols (*e.g*., propylene glycol), polyalkylone glycols (*e.g*., polyethylene glycol) oils, alcohol, slightly acidic buffers between pH 4 and pH 6 (*e.g*., acetate, citrate, ascorbate at between about 5.0 mM to about 50.0 mM), *etc.* Additionally, flavoring agents, preservatives, coloring agents, bile salts, acylcarnitines and the like may be added. For buccal administration, the pharmaceutical compositions may take the form of tablets, lozenges, *etc.* formulated in conventional manner.

When a compound of the invention is acidic or basic, it may be included in any of the above-described formulations as the free acid or free base, a pharmaceutically acceptable salt, a solvate or hydrate. Pharmaceutically acceptable salts substantially retain the activity of the free acid or base, may be prepared by reaction with bases or acids and tend to be more soluble in aqueous and other protic solvents than the corresponding free acid or base form.

### Therapeutic Doses

A compound of the invention and/or pharmaceutical composition thereof, will generally be used in an amount effective to achieve the intended purpose. For use to treat or prevent the above diseases or disorders the compounds of the invention and/or pharmaceutical compositions thereof, are administered or applied in a therapeutically effective amount.

The amount of a compound of the invention and/or pharmaceutical composition thereof that will be effective in the treatment of a particular disorder or condition disclosed herein will depend on the nature of the disorder or condition, and can be determined by standard clinical techniques known in the art and by doctors skilled in treating or preventing a particular disease or disorder. In addition, *in vitro* or *in vivo* assays may optionally be employed to help identify optimal dosage ranges. The amount of a compound of the invention and/or pharmaceutical composition thereof administered will, of course, be dependent on, among other factors, the subject being treated, the weight of the subjects, the severity of the affliction, the manner of administration and the judgment of the prescribing physician.

For example, the dosage may be delivered in a pharmaceutical composition by a single administration, by multiple applications or controlled release. The compounds of the invention may be delivered by oral sustained release administration. Preferably, in this example, the compounds of the invention are administered twice per day (more preferably, once per day). Dosing may be repeated intermittently, may be provided alone or in combination with other drugs and may continue as long as required for effective treatment of the disease state or disorder.

Suitable dosage ranges for oral administration are dependent on the nature of the compounds of the invention administered (*e.g*., whether the theaflavins are administered together or whether theaflavin, theaflavin-3-gallate, theaflavin-3'-gallate or theaflavin-3,3'-digallate are administered, each as a separate compound) but are generally about 0.001 mg to about 200 mg of a compound of the invention per kilogram body weight. In one embodiment, the dosage range is between about 0.1 mg/kg to about 5 mg/kg. Dosage ranges may be readily determined by methods known to the artisan of ordinary skill. Effective doses may be extrapolated from dose-response curves derived from *in vitro* or animal model test systems. Such animal models and systems are well-known in the art.

The compounds of the invention are preferably assayed *in vitro* and *in vivo,* for the desired therapeutic or prophylactic activity, prior to use in humans. For example, *in vitro* assays can be used to determined whether administration of a specific compound of the invention or a combination of compounds of the invention is preferred. The compounds of the invention may also be demonstrated to be effective and safe using animal model systems.

Preferably, a therapeutically effective dose of a compound of the invention and/or pharmaceutical composition thereof described herein will provide therapeutic benefit without causing substantial toxicity. Toxicity of compounds of the invention and/or pharmaceutical compositions thereof may be determined using standard pharmaceutical procedures and may be readily ascertained by the skilled artisan. The dose ratio between toxic and therapeutic effect is the therapeutic index. A compound of the invention and/or pharmaceutical composition thereof will preferably exhibit particularly high therapeutic indices in treating disease and disorders. The dosage of a compound of the invention and/or pharmaceutical composition thereof described herein will preferably be within a range of circulating concentrations that include an effective dose with little or no toxicity.

### Combination Therapy

The compounds of the invention and/or pharmaceutical compositions thereof can be used in combination therapy with at least one other therapeutic agent. The compound of the invention and/or pharmaceutical composition thereof and the therapeutic agent can act additively or, more preferably, synergistically. A compound of the invention and/or a pharmaceutical composition thereof may be administered concurrently with the administration of another therapeutic agent. A compound of the invention and/or pharmaceutical composition thereof may be administered prior or subsequent to administration of another therapeutic agent.

The compounds of the invention and/or pharmaceutical compositions thereof can be used in combination therapy with other agents used to treat or prevent hyperlipidemia, coronary heart disease, apoplexy, arthesclerotic cardiovascular diseases, AIDES, diabetes, oxidated-low density lipoprotein level, von Willebeand's disease, leukopenia, cerebral infarction, dementia and physical disorder and fatty liver.

### Diet Supplement Compositions

The diet supplement compositions described herein typically contain one or more compounds of the invention, preferably in purified form, together with a suitable amount of a diet supplement vehicle, so as to provide the form for proper administration to a user.

### Examples

The invention is further defined by reference to the following examples, which describe in detail, preparation of compounds of the invention and methods for assaying for biological activity. It will be apparent to those skilled in the art that many modifications, both to materials and methods, may be practiced without departing from the scope of the invention.

### Example 1: Preparation and Isolation of the Mixture of Theaflavin, Theaflavin-3-gallate, Theaflavin-3'-gallate and Theaflavin-3,3' digallate

Green tea leaves (100 kg) obtained from FML Tea Trading Co., Ltd., Xiamen, China and 0.15 M phosphate buffer (1000 L, pH 6.4) was added to a slurry fermenter maintained at 30°C. Oxygen was added to the fermenter at a flow rate of 20 L/sec until the total oxygen concentration was 22%, to initiate fermentation. The reaction mixture was stirred and fermented for about 1 hour under a 22% oxygen atmosphere.

The reaction mixture was centrifuged and the supernatant decanted. The supernatant was filtered to remove solids. About 1000 L of ethyl acetate was added to the aqueous filtrate, which terminated fermentation and the layers were separated. The organic layer was washed three times with 1000 L of 5% NaHCO₃ and ethyl acetate was removed in *vacuo.* Then, the residue was dissolved in a small portion of 40% ethyl acetate/hexanes, loaded onto a column, which contained 150 kg of silica gel and eluted with 40% ethyl acetate/hexanes to provide a mixture of theaflavin, theaflavin-3-gallate, theaflavin-3'-gallate and theaflavin-3,3' digallate (typically, between about 50 kg to 60 kg) in 99.3% purity as determined by reverse phase High Pressure Reverse Phase Chromatography (HPLC). The mixture of theaflavins comprised 46.8% theaflavin, 18.5% theaflavin-3-gallate, 13.7% theaflavin-3'-gallate and 20.3% theaflavin-3,3' digallate as determined by HPLC-MS.

### Example 2: Preparation and Isolation of Theaflavin, Theaflavin-3-gallate, Theaflavin-3'-gallate and Theaflavin-3,3' digallate, Each as a Separate Compound

Tea polyphenol (100 kg), which contains 98.6%, catechins (45.6% epigallocatechin gallate), (Yuan Xiang Biopharm Company, Ltd., Shanghai, China), 1000 L of 0.15 M phosphate buffer (pH 6.4) and 10 kg of polyphenol oxidase was added to a slurry fermenter at 25 °C with stirring. Oxygen at a flow rate of 20 L/sec was added to the fermenter until the total oxygen concentration was 22%, to initiate fermentation. The reaction mixture was stirred and fermented for about 40 minutes under a 22% oxygen atmosphere.

The reaction mixture was centrifuged and the supernatant decanted. The supernatant was filtered to remove solids. About 1000 L of ethyl acetate was added to the aqueous filtrate, which terminated fermentation and the layers were separated. The organic layer was washed three times with 1000 L of 5% NaHCO₃ and ethyl acetate was removed in *vacuo.* Then, the residue was dissolved in a small portion of 40% ethyl acetate/hexanes and divided into 20 separate portions. Each portion was then loaded onto a column containing 150 kg of silica gel and eluted with 40% ethyl acetate/hexanes to provide theaflavin (25 kg), theaflavin-3-gallate (9kg), theaflavin-3'-gallate(6 kg) and theaflavin-3,3' digallate (10 kg), each as a separate compound. The theaflavins were then further purified by recrystallization from ethanol. The final purity of theaflavin, theaflavin-3-gallate, theaflavin-3'-gallate and theaflavin-3,3' digallate, as determined by HPLC was 99.1%, 98.7%, 97.8% and 99.3%, respectively. The structure of theaflavin, theaflavin-3-gallate, theaflavin-3'-gallate and theaflavin-3,3' digallate was determined by HPLC-MS.

### Example 3: Preparation of Soft Gels of Theaflavins

Beeswax (2 kg) was added to olive oil (500 kg) at 80 °C with stirring for 30 minutes and cooled to 40 °C. A mixture of theaflavins (100 kg), prepared via the method of Example 1, was mixed thoroughly into the above mixture of beeswax and olive oil. The resulting mixture of theaflavins, beeswax and olive oil were placed into a gel comprised of gelatin powder to prepare the soft gel capsules. Each soft gel capsule is about 700mg of which about 75mg are the mixture of theaflavins.

### Example 4: Use of Theaflavins to Elevate Lipid Profile in Rats

Male Sprague-Dawley rats weighing 100 g to 130 g were divided into nine groups of ten rats. The animals were individually housed in stainless-steel cages with screen bottoms in a room maintained at between 22 °C and 24 °C. They were fed stationary feedstuff and water as follows as described in Table 1. In addition the rats in Group TF1-1, TF1-2 or TF1-3 were fed pure theaflavin in a dosage of 1 mg/kg/day, 2 mg/kg/day and 4 mg/kg/day, respectively. The animals in group TF2A-2, TF2B-2 or RF3-2 were fed pure theaflavin-3-gallate, theaflavin-3'-gallate and theaflavin-3,3'-digallate, respectively in a dosage of 2 mg/kg/day. The animals in group CA-1 and CA-2 were fed catechins in a dosage of 1 mg/kg/day or 2 mg/kg/day, respectively.

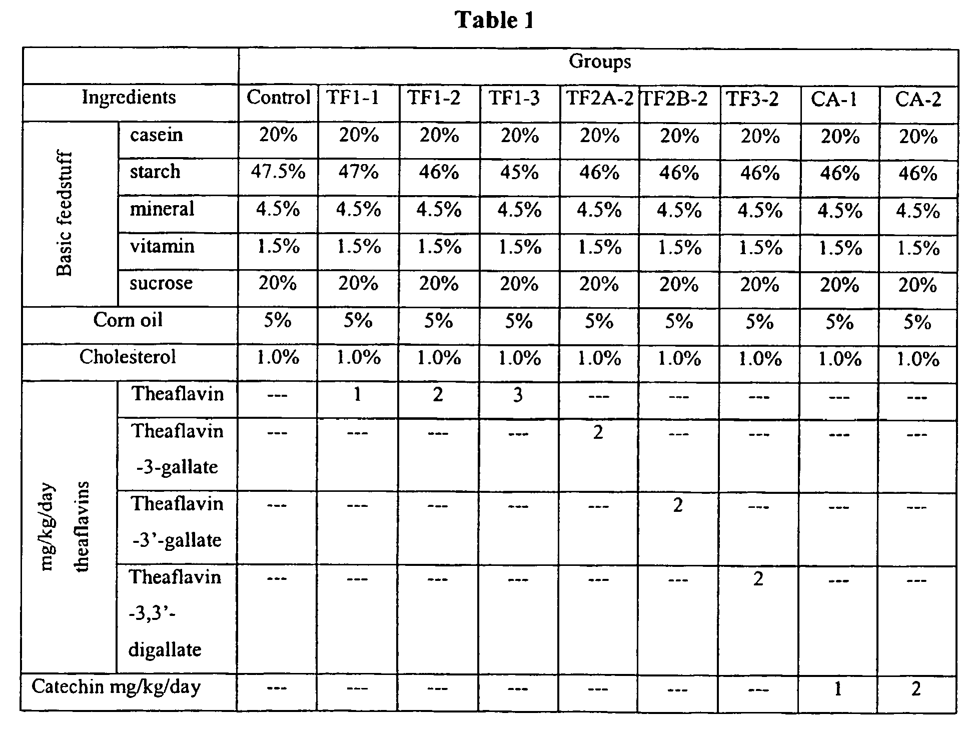

The Sprague Dawley rats were fed the above diets for eight weeks before sampling. The rats were not fed for 12 hours prior to sampling. The rats were given either theaflavin, theaflavin-3-gallate, theaflavin-3'-gallate, theaflavin-3,3'-digallate or catechin one hour in advance of collecting the blood sample.

**Table 2 Lipids profile of rats after taking catechin or theaflavins**

| | Groups | | | | | | | | |
|---|---|---|---|---|---|---|---|---|---|
| | Control | TF1-1 | TF1-2 | TF1-3 | TF2A-2 | TF2B-2 | TF3-2 | CA-1 | CA-2 |
| TG | 120.2 ±14.4 | 108.1 ±13.8² | 97.4 ±11.6¹ | 87.7 ±12.2¹ | 95.7 ±10.5¹ | 96.2 ±10.2¹ | 95.36 ±9.2¹ | 113.9 ±12.0³ | 106.7 ±10.5² |
| TC | 131.3 ±10.0 | 116.3 ±8.2¹ | 101.3 ±7.9¹ | 90.8 ±7.1¹ | 97.3 ±8.4¹ | 97.8 ±8.5¹ | 95.5 ±7.4¹ | 123.1 ±8.1³ | 113.2 ±11.1³ |
| LDL | 54.9 ±3.4 | 45.6 ±3.1¹ | 34.6 ±3.7¹ | 25.3 ±2.2¹ | 32.2 ±3.7¹ | 32.4 ±3.3¹ | 31.2 ±4.1¹ | 49.5 ±3.3³ | 44.1 ±2.43 |
| HDL | 48.3 ±1.7 | 48.8 ±1.7³ | 49.2 ±2.1³ | 49.4 ±1.9³ | 49.2 ±1.9³ | 49.3 ±2.7³ | 49.3 ±1.9³ | 48.2 ±2.2³ | 48.4 ±1.9³ |

| | | | | | | | | | |
|---|---|---|---|---|---|---|---|---|---|
| ¹ P<0.01, compared to the control group ² P<0.05, compared to the control group ³ There is no significant difference between the control and medicine group | | | | | | | | | |

As shown in Table 2, the rats in the theaflavin groups had greatly decreased dose dependent plasma levels of total cholesterol ("TC"), low density lipoprotein-cholesterol ("LDL-C") and triglyceride ("TG") when compared with to the control group. Theaflavin, theaflavin-3-gallate, theaflavin-3'-gallate, theaflavin-3,3'-digallate were more effective in reducing plasma levels of TC, LDL-C and TG than catechin.

### Example 5: Effect of a Mixture of Theaflavins on Subjects with Mild to Moderate Hyperlipidemia

Choleve^{™} is a mixture of theaflavin, theaflavin-3-gallate, theaflavin-3'-gallate and theaflavin-3,3'-digallate prepared as described in Example 1. The clinical trial was a double-blind, randomized, placebo-controlled, parallel-group trial, which lasts for 12 weeks.

Patients were 19 years of age and older with mild to moderate hyperlipidemia (LDL-C 130-190 mg/dL) on a low fat diet and were recruited from outpatient clinics in six hospitals in China. Patients were excluded if their baseline triglycerides were 350 mg/dL or greater or if they had uncontrolled hypertension (≥160/95 mm Hg). Patients with active pulmonary, hematologic, hepatic, gastrointestinal or renal disease, pre-malignant or malignant disease, diabetes, thyroid dysfunction, a history of coronary heart disease or other atherosclerotic disease were also excluded. Patients with pathological values among routine clinical chemistry or hematological parameters or if they consumed > 32% of daily energy from fat or had a body mass index ≥35 were also excluded. Subjects were also excluded if they were taking any lipid lowering medications or drugs that might interfere with lipid metabolism, cardiac or other vasoactive medications including antihypertensive drugs, thyroid hormones, oral contraceptives, cyclic hormone replacement therapy, dietary supplements (e.g., fish oils, niacin at doses >400mg/day, dietary fiber supplements), or antioxidants and they were prohibited from taking these medications during the course of the study. Patients were required to maintain a stable diet and level of physical activity during a 12-week study.

The inclusion and exclusion criteria were met by two hundred and forty patients. After randomization, clinic visits were scheduled in the morning of weeks 4, 8, and 12 after a 12-hour fast. Parameters measured at each visit included body weight, blood pressure and heart rate. Subjects were asked to record study capsules taken, concomitant medications, and any adverse events in subject diaries. Study capsules were dispensed at baseline, week 4, and week 8. Nutrient intake was assessed using 3-day food records completed by the subjects prior to baseline and after eight weeks of treatment. Lipid and lipoprotein concentrations were measured at week -2, week 0, week 4 and week 12. Lipids and lipoproteins were analyzed enzymatically using standard laboratory procedures. The results, which include serum chemistry and hematology tests, were tested on an automatic biochemical instrument (Sybchron Clinical System LX 20, Beckman Coulter, Inc., Shanghai Representative Office Room 3203-3205, Hong Kong Plaza, South Building, 283 Huai Hai Zhong Road, Shanghai, China 200021). Platelet aggregation was induced by adenosine diphosphate and the rate of platelet aggregation was determined by the optical density measurements at 915 nm.

Two hundred forty subjects were randomized into the study, and two hundred and twenty subjects completed the trial. The baseline characteristics are presented in Table 1. All subjects were Asian with 95% of subjects in the treatment group and 88% in the placebo group finishing the study. Fourteen subjects failed to return, five subjects were discontinued from the study at their request and one subject was terminated for protocol violation.

After four weeks and twelve weeks of oral administration of Choleve^{™}, the serum LDL-C level in the subjects decreased by 9.6% (4.08mmol/1-3.68mmol/L) and 16.4% (4.08mmol/l-3.40mmol/l) compared to the base line at start (P=0.0001) (see Tables 3 and 4). At the same time, Choleve^{™} caused 6.7% and 11.3% decrease in the serum TC level (P=0.0001). On the other hand, the LDL-C and TC levels in the placebo group showed no significant changes after four weeks and twelve weeks. However, there are no significant changes in the TG and high density lipoprotein ("HDL") levels between Choleve^{™} and placebo group after 12 weeks treatment.

**Table 3. Fasting serum lipid parameters (mmol/L) after 4 week and 12 weeks' treatment**

| | **0 weeks** | | **4 weeks** | | **12 weeks** | |
|---|---|---|---|---|---|---|
| | **Choleve^{™} (n=114)** | **Placebo (n=109)** | **Choleve^{™} (n=114)** | **Placebo (n=109)** | **Choleve^{™} (n=114)** | **Placebo (n=109)** |
| **TC** | 6.31±0.58 | 6.18±0.64 | 5.87±0.68^{2,3} | 6.18±0.80 | 5.58±0.70^{2,3} | 6.12±0.64 |
| **LDL-c** | 4.08±0.42 | 4.00±0.46 | 3.68±0.55^{2,3} | 4.06±0.65 | 3.40±0.56^{2,3} | 4.00±0.55 |
| **HDL-c⁴** | 1.43±0.32 | 1.45±0.36 | 1.40±0.32 | 1.42±0.35 | 1.42±0.29 | 1.41±0.32 |
| **TG⁵** | 2.10±0.86 | 1.93±0.82 | 2.08±0.97 | 2.00±0.82 | 2.07±1.02 | 1.90±0.79 |

| | | | | | | |
|---|---|---|---|---|---|---|
| ¹T=SD ²P=0.0001 compared to the baseline ³ P-0.001 compared to the baseline, the baseline value as covariance ^{4,5} there is no significant difference between the active and placebo group | | | | | | |

**Table 4. Mean percent change from baseline in lipid parameters¹ (χ±SE)**

| | **4 weeks** | | **12 weeks** | |
|---|---|---|---|---|
| | **Choleve^{™} (n=114)** | **Placebo (n=109)** | **Choleve^{™} (n=114)** | **Placebo (n=109)** |
| **TC (%)²** | -6.70±0.84 | 0.05±0.87 | -11.34±0.89 | -0.72±0.77 |
| **LDL-c(%)³** | -9.58±1.09 | 1.75±1.29 | -16.4±1.10 | 0.26±1.24 |
| **HDL-c(%)⁴** | 0.35±2.10 | -0.36±1.65 | 2.30±2.06 | -0.69±1.59 |
| **TG(%)⁵** | 0.51±2.58 | 11.79±4.82 | 2.58±3.52 | 5.58±3.76 |

| | | | | |
|---|---|---|---|---|
| ¹(χ±SEM ^{2,3} P=0.0001 compared to the baseline ^{4,5} there is no significant difference between the active and placebo group | | | | |

Table 5 illustrates that Choleve™ had no effect to the platelet aggregation.

**Table 5. PLAG(%) after 4 week and 12 weeks' duration**

| | **4 weeks** | | **12 weeks** | |
|---|---|---|---|---|
| | **Choleve^{™}** (n=114) | **Placebo (n=109)** | **Choleve^{™} (n=114)** | **Placebo (n=109)** |
| **PLAG%(1min)** | 40.2±16.1 | 37.5±14.0 | 36.8±15.6 | 40.3±16.1 |
| **PLAG%(Tmax)** | 56.2±20.6 | 57.3±19.1 | 56.5±23.0 | 57.4±19.1 |

After twelve weeks of oral administration of Choleve^{™}, the extract decreased serum total cholesterol and LCL-C by 11.3% and 16.4%, respectively, as shown above, which indicates the potential of Choleve^{™} in treating and preventing coronary heart disease. No adverse reactions to Choleve^{™} occurred during this clinical trial, which demonstrates the safety of Choleve^{™}.

## Claims

1. A method of making a mixture of theaflavin, theaflavin-3-gallate, theaflavin-3'-gallate and theaflavin 3,3'-digallate, said method comprising the steps of:
contacting green tea leaves with an aqueous buffer having a pH of 5.0 to 7.0 to form a reaction mixture, wherein the ratio of green tea leaves to aqueous buffer is preferably between 1:0.5 and 1:20 on a kilogram to liter basis;
contacting the reaction mixture with oxygen to begin fermentation;
fermenting the reaction mixture for 0.5 to 1.5 hours;
terminating fermentation; and
separating the reaction mixture from the mixture of theaflavin, theaflavin-3-gallate, theaflavin-3'-gallate and theaflavin 3,3' digallate.

2. A method according to claim 1, wherein the separating step further comprises:
optionally removing the solids from the reaction mixture;
contacting the reaction mixture with an organic solvent to dissolve the mixture of theaflavin, theaflavin-3-gallate, theaflavin-3'-gallate and theaflavin 3,3' digallate;
contacting the solvent with dilute aqueous base;
separating the solvent from the base;
contacting the solvent with a chromatographic medium, preferably silica gel; and
eluting the mixture of theaflavin, theaflavin-3-gallate, theaflavin-3'-gallate and theaflavin 3,3' digallate from the chromatographic medium.

3. A method according to claim 1 or 2, wherein the ratio of green tea leaves to aqueous buffer is between 1:0.5 and 1:20 on a kilogram to liter basis, the aqueous buffer is 0.15 M phosphate buffer at pH 6.4, and the reaction mixture is fermented between 15°C and 80°C for 0.5 to 1.5 hours while being agitated.

4. A method for making theaflavin, theaflavin-3-gallate, theaflavin-3'-gallate and theaflavin-3,3'-digallate, each as a separate compound, said method comprising the steps of:
contacting tea polyphenols with an aqueous buffer having a pH of 5.0 to 7.0 and polyphenol oxidase to form a reaction mixture, wherein preferably the ratio of tea polyphenol to buffer is between 1:0.1 and 1:2 on a weight to weight basis;
contacting the reaction mixture with oxygen to begin fermentation;
fermenting the reaction mixture for 0.5 to 1.5 hours;
terminating fermentation; and
separating the reaction mixture to provide theaflavin, theaflavin-3-gallate, theaflavin-3'-gallate or theaflavin-3,3'-digallate, each as a separate compound, wherein each is preferably more than 97% pure.

5. A method according to claim 4, wherein the separating step further comprises:
optionally removing solids from the reaction mixture;
contacting the reaction mixture with an organic solvent;
contacting the solvent with dilute aqueous base;
separating the solvent from the base; and
contacting the solvent with a chromatographic medium, preferably silica gel; and
eluting the theaflavin, theaflavin-3-gallate, theaflavin-3'-gallate and theaflavin 3,3' digallate, each as a single compound, from the chromatographic medium.

6. A method according to claim 4 or 5, wherein the ratio of tea polyphenol to buffer is between 1:0.5 and 1:20 on a kilogram to liter basis, the buffer is 0.15 M phosphate buffer at pH 6.4, and the reaction mixture is fermented at between about 15°C and about 80°C for 0.5 to 1.5 hours.

7. A pharmaceutical composition comprising:
(a) as the pharmaceutically active agent, an isolated mixture of theaflavin, theaflavin-3-gallate, theaflavin-3'-gallate, and theaflavin-3,3'-digallate; and
(b) one or more physiologically acceptable excipients, carriers, or diluents;
wherein the composition is for administration of the mixture (a) at a dose in the range of 0.001 mg to 200mg/kg body weight.

8. A pharmaceutical composition according to claim 7 wherein the theaflavin mixture is present at a level of between 5% and 50% (w/w) in the composition.

9. A pharmaceutical composition comprising:
(a) as the pharmaceutically active agent, a mixture of theaflavin, theaflavin-3-gallate, theaflavin-3'-gallate, and theaflavin-3,3'-digallate; and
(b) one or more physiologically acceptable excipients, carriers, or diluents;
wherein the theaflavin mixture is present at a level of between 5% and 50% (w/w) in the composition.

10. A pharmaceutical composition according to claim 9 wherein the composition is for administration of the mixture (a) at a dose in the range of 0.001mg to 200mg/kg body weight.

11. A pharmaceutical composition consisting of:
(a) as the pharmaceutically active agent, a mixture of theaflavin, theaflavin-3-gallate, theaflavin-3'-gallate, and theaflavin-3,3'-digallate; and
(b) one or more physiologically acceptable excipients, carriers or diluents;
wherein the composition is for administration of the mixture (a) at a dose in the range of 0.001 mg to 200mg/kg body weight.

12. A pharmaceutical composition according to claim 11 wherein the theaflavin mixture is present at a level of between 5% and 50% (w/w) in the composition.

13. A pharmaceutical composition according to any of claims 7 to 12 wherein the composition is for administration of the mixture (a) at a dose in the range of 0.1 mg to 5mg/kg body weight.

14. A pharmaceutical composition according to any of claims 7 to 13 wherein the amount of the mixture (a) per dose is 70 to 210mg.

15. A pharmaceutical composition according to any of claims 7 to 14 wherein the composition is for single administration dosage delivery.

16. A pharmaceutical composition according to any one of claims 7 to 15 which is in the form of a capsule.

17. A pharmaceutical composition according to claim 16 which is in the form of a soft gel capsule, and wherein the composition comprises the theaflavin mixture, beeswax, olive oil and gelatin.

18. A dietary supplement composition consisting of:
a mixture of theaflavin, theaflavin-3-gallate, theaflavin-3'-gallate, and theaflavin-3,3'-digallate;
and a suitable diet supplement vehicle;
wherein the composition is for administration of the mixture (a) at a dose in the range of 0.001 mg to 200mg/kg body weight.

19. A mixture of theaflavin, theaflavin-3-gallate, theaflavin-3'-gallate, and theaflavin-3,3'-digallate for use in a method of treating or preventing hyperlipidemia,

20. A mixture of theaflavin, theaflavin-3-gallate, theaflavin-3'-gallate, and theaflavin-3,3'-digallate for use in a method of decreasing low density lipoprotein-cholesterol (LDL-c) levels without significantly changing high density lipoprotein-cholesterol (HDL-c) levels.

21. Use of a mixture of theaflavin, theaflavin-3-gallate, theaflavin-3'-gallate, and theaflavin-3,3'-digallate in the manufacture of a medicament for treating or preventing hyperlipidemia.

22. Use of a mixture of theaflavin, theaflavin-3-gallate, theaflavin-3'-gallate, and theaflavin-3,3'-digallate in the manufacture of a medicament for decreasing LDL-c levels without significantly changing HDL-c levels.

## Patentansprüche

1. Verfahren zur Herstellung eines Gemisches aus Theaflavin, Theaflavin-3-gallat, Theaflavin-3'-gallat und Theaflavin-3,3'-digallat, wobei das Verfahren folgende Schritte umfasst:
das Kontaktieren von Grünteeblättern mit einem wässrigen Puffer mit einem pH von 5,0 bis 7,0, um ein Reaktionsgemisch zu bilden, worin das Verhältnis von Grünteeblättern zu wässrigem Puffer vorzugsweise zwischen 1:0,5 und 1:20 auf einer Kilogramm-zu-Liter-Basis liegt;
das Kontaktieren des Reaktionsgemisches mit Sauerstoff, um die Fermentation zu i-nitrieren;
das Fermentieren des Reaktionsgemisches für eine Zeitspanne von 0,5 bis 1,5 Stunden;
das Beenden der Fermentation; sowie
das Trennen des Reaktionsgemisches von dem Gemisch aus Theaflavin, Theaflavin-3-gallat, Theaflavin-3'-gallat und Theaflavin-3,3'-digallat.

2. Verfahren nach Anspruch 1, worin der Trennungsschritt weiters Folgendes umfasst:
gegebenenfalls das Entfernen der Feststoffe aus dem Reaktionsgemisch;
das Kontaktieren des Reaktionsgemisches mit einem organischen Lösungsmittel, um das Gemisch aus Theaflavin, Theaflavin-3-gallat, Theaflavin-3'-gallat und Theaflavin-3,3'-digallat aufzulösen;
das Kontaktieren des Lösungsmittels mit verdünnter wässriger Base;
das Trennen des Lösungsmittels von der Base;
das Kontaktieren des Lösungsmittels mit einem chromatographischen Medium, vorzugsweise Sllicagel; sowie
das Eluieren des Gemisches aus Theaflavin, Theaflavin-3-gallat, Theaflavin-3'-gallat und Theaflavin-3,3'-digallat aus dem chromatographischen Medium.

3. Verfahren nach Anspruch 1 oder 2, worin das Verhältnis an Grünteeblättern zu wässrigem Puffer zwischen 1:0,5 und 1:20 auf einer Kilogramm-zu-Liter-Basis liegt, der wässrige Puffer 0,15 M Phosphatpuffer mit einem pH von 6,4 ist und das Reaktionsgemisch für eine Zeitspanne von 0,5 bis 1,5 Stunden zwischen 15°C und 80 °C unter Rühren fermentiert wird.

4. Verfahren zur Herstellung von Theaflavin, Theaflavin-3-gallat, Theaflavin-3'-gallat und Theaflavin-3,3'-digallat, jeweils als separate Verbindung, wobei das Verfahren folgende Schritte umfasst:
das Kontaktieren der Tee-Polyphenole mit einem wässrigen Puffer mit einem pH von 5,0 bis 7,0 und Polyphenol-Oxidese, um ein Reaktionsgemisch zu bilden, worin das Verhältnis von Tee-Polyphenol zu Puffer vorzugsweise zwischen 1:0,1 und 1:2 auf einer Gewicht-zu-Gewicht-Basis liegt;
das Kontaktieren des Reaktionsgemisches mit Sauerstoff, um die Fermentation zu i-nitrieren;
das Fermentieren des Reaktionsgemisches für eine Zeitspanne von 0,5 bis 1,5 Stunden;
das Beenden der Fermentation; sowie
das Trennen des Reaktionsgemisches, um Theaflavin, Theaflavin-3-gallat, Theaflavin-3'-gallat oder Theaflavin-3,3'-digallat, jeweils als separate Verbindung, bereitzustellen, wobei jede vorzugsweise zu mehr als 97 % rein ist.

5. Verfahren nach Anspruch 4, worin der Trennungsschritt weiters Folgendes umfasst:
gegebenenfalls das Entfernen der Feststoffe aus dem Reaktionsgemisch;
das Kontaktieren des Reaktionsgemisches mit einem organischen Lösungsmittel;
das Kontaktieren des Lösungsmittels mit verdünnter wässriger Base;
das Trennen des Lösungsmittels von der Base;
das Kontaktieren des Lösungsmittels mit einem chromatographischen Medium, vorzugsweise Silicagel; sowie
das Eluieren von Theaflavin, Theaflavin-3-gallat, Theaflavin-3'-gallet und Theaflavin-3,3'-digallat, jeweils als einzelne Verbindung, aus dem chromatographischen Medium.

6. Verfahren nach Anspruch 4 oder 5, worin das Verhältnis von Tee-Polyphenol zu Puffer zwischen 1:0,5 und 1:20 auf einer Kilogramm-zu-Liter-Basis liegt, der Puffer 0,15 M Phosphatpuffer mit einem pH von 6,4 ist und das Reaktionsgemisch für eine Zeitspanne von 0,5 bis 1,5 Stunden bei zwischen etwa 15 °C und etwa 80 °C fermentiert wird.

7. Pharmazeutische Zusammensetzung, umfassend:
(a) ein isoliertes Gemisch aus Theaflavin, Theaflavin-3-gallat, Theaflavin-3'-gallat und Theaflavin-3,3'-digallat als pharmazeutisch aktives Mittel; sowie
(b) einen oder mehrere physiologisch annehmbare Exzipienten, Träger oder Verdünnungsmittel;
worin die Zusammensetzung zur Verabreichung des Gemisches (a) in einer Dosis im Bereich von 0,001 mg bis 200 mg/kg Körpergewicht dient.

8. Pharmazeutische Zusammensetzung nach Anspruch 7, worin das Theaflavin-Gemisch in einer Menge zwischen 5 und 50 Gew.-% in der Zusammensetzung vorliegt.

9. Pharmazeutische Zusammensetzung, umfassend:
(a) ein Gemisch aus Theaflavin, Theaflavin-3-gallat, Theaflavin-3'-gallat und Theaflavin-3,3'-digallat als pharmazeutisch aktives Mittel; sowie
(b) einen oder mehrere physiologisch annehmbare Exzipienten, Träger oder Verdünnungsmittel;
worin das Theaflavin-Gemisch in einer Menge zwischen 5 und 50 Gew.-% In der Zusammensetzung vorliegt.

10. Pharmazeutische Zusammensetzung nach Anspruch 9, worin die Zusammensetzung zur Verabreichung des Gemisches (a) in einer Dosis im Bereich von 0,001 mg bis 200 mg/kg Körpergewicht dient.

11. Pharmazeutische Zusammensetzung, bestehend aus:
(a) einem Gemisch aus Theaflavin, Theaflavin-3-gallat, Theaflavin-3'-gallat und Theaflavln-3,3'-digallat als pharmazeutisch aktivem Mittel; sowie
(b) einem oder mehreren physiologisch annehmbaren Exzipienten, Trägern oder Verdünnungsmitteln;
worin die Zusammensetzung zur Verabreichung des Gemisches (a) in einer Dosis im Bereich von 0,001 mg bis 200 mg/kg Körpergewicht dient.

12. Pharmazeutische Zusammensetzung nach Anspruch 11, worin das Theaflavin-Gemisch in einer Menge zwischen 5 und 60 Gew.-% in der Zusammensetzung vorliegt.

13. Pharmazeutische Zusammensetzung nach einem der Ansprüche 7 bis 12, worin die Zusammensetzung zur Verabreichung des Gemisches (a) in einer Dosis im Bereich von 0,1 mg bis 5 mg/kg Körpergewicht dient.

14. Pharmazeutische Zusammensetzung nach einem der Ansprüche 7 bis 13, worin die Menge des Gemisches (a) pro Dosis 70 bis 210 mg beträgt.

15. Pharmazeutische Zusammensetzung nach einem der Ansprüche 7 bis 14, worin die Zusammensetzung zu einer Dasisanlieferung durch einmalige Verabreichung dient.

16. Pharmazeutische Zusammensetzung nach einem der Ansprüche 7 bis 15, die in Form einer Kapsel vorliegt.

17. Pharmazeutische Zusammensetzung nach Anspruch 16, die in Form einer wiechen Gel-Kapsel vorliegt und worin die Zusammensetzung das Theaflavin-Ge-, misch, Bienenwachs, Olivenöl und Gelatine umfasst.

18. Nahrungsergänzungsmittel-Zusammensetzung, bestehend aus:
einem Gemisch aus Theaflavin, Theaflavin-3-gallat, Theaflavin-3'-gallat und Theaflavin-3,3'-digallat;
sowie einem geeigneten Nahrungsergänzungsmittel-Vehikel;
worin die Zusammensetzung zur Verabreichung des Gemisches (a) in einer Dosis im Bereich von 0,001 mg bis 200 mg/kg Körpergewicht dient.

19. Gemisch aus Theaflavin, Theaflavin-3-gallat, Theaflavin-3'-gallat und Theaflavin-3,3'-digallat zur Verwendung in einem Verfahren zur Behandlung oder zur Prävention von Hyperlipidämie.

20. Gemisch aus Theaflavin, Theaflavln-3-gallat, Theaflavin-3'-gallat und Theaflavin-3,3'-digallat zur Verwendung in einem Verfahren zur Verringerung von Lipoprotein-Cholesterin-Spiegeln niedriger Dichte (LDL-c-Spiegeln), ohne Lipoprotein-Cholesterin-Spiegel hoher Dichte (HDL-c-Spiegel) signifikant zu verändern.

21. Verwendung eines Gemisches aus Theaflavin, Theaflavin-3-gallat, Theaflavin-3'-gallat und Theaflavln-3,3'-digallat zur Herstellung eines Medikaments zur Behandlung oder Prävention von Hyperlipidämie.

22. Verwendung eines Gemisches aus Theaflavin, Theaflavin-3-gallat, Theaflavin-3'-gallat und Theaflavin-3,3'-dlgallat zur Herstellung eines Medikaments zur Verringerung von LDL-c-Spiegeln, ohne HDL-c-Spiegel signifikant zu verändern.

## Revendications

1. Procédé de préparation d'un mélange de théaflavine, de 3-gallate de théaflavine, de 3'-gallate de théaflavine et de 3,3'-digallate de théaflavine, ledit procédé comprenant les étapes consistant à :
mettre en contact des feuilles de thé vert avec un tampon aqueux ayant un pH de 5,0 à 7,0 pour former un mélange de réaction, le rapport des feuilles de thé vert sur le tampon aqueux étant de préférence compris entre 1:0,5 et 1:20 sur une base kilogramme sur litre ;
mettre en contact le mélange de réaction avec de l'oxygène pour commencer une fermentation ;
faire fermenter le mélange de réaction pendant 0,5 à 1,5 heure ;
terminer la fermentation ; et
séparer le mélange de réaction du mélange de théaflavine, de 3-gallate de théaflavine, de 3'-gallate de théaflavine et de 3,3'-digallate de théaflavine.

2. Procédé selon la revendication 1, dans lequel l'étape de séparation comprend en outre les sous-étapes consistant à :
retirer facultativement les solides du mélange de réaction ;
mettre en contact le mélange de réaction avec un solvant organique pour dissoudre le mélange de théaflavine, de 3-gallate de théaflavine, de 3'-gallate de théaflavine et de 3,3'-digallate de théaflavine,
mettre en contact le solvant avec une base aqueuse diluée ;
séparer le solvant de la base ;
mettre en contact le solvant avec un milieu chromatographique, de préférence un gel de silice ; et
éluer le mélange de théaflavine, de 3-gallate de théaflavine, de 3'-gallate de théaflavine et de 3,3'-digallate de théaflavine du milieu chromatographique.

3. Procédé selon la revendication 1 ou 2, dans lequel le rapport des feuilles de thé vert sur le tampon aqueux est compris entre 1:0,5 et 1:20 sur une base kilogramme sur litre, le tampon aqueux étant un tampon au phosphate à 0,15 M au pH 6,4, et le mélange de réaction est fermenté entre 15 °C et 80 °C pendant 0,5 à 1,5 heure tout en étant agité.

4. Procédé de préparation de théaflavine, de 3-gallate de théaflavine, de 3'-gallate de théaflavine et de 3,3'-digallate de théaflavine, chacun sous forme de composé séparé, ledit procédé comprenant les étapes consistant à :
mettre en contact des polyphénols de thé avec un tampon aqueux ayant un pH de 5,0 à 7,0 et une polyphénol oxydase pour former un mélange de réaction, où de préférence le rapport polyphénol de thé sur tampon est compris entre 1:0,1 et 1:2 sur une base poids sur poids ;
mettre en contact le mélange de réaction avec de l'oxygène pour commencer une fermentation ;
faire fermenter le mélange de réaction pendant 0,5 à 1,5 heure ;
terminer la fermentation ; et
séparer le mélange de réaction pour former de la théaflavine, du 3-gallate de théaflavine, du 3'-gallate de théaflavine ou du 3,3'-digallate de théaflavine, chacun sous forme de composé séparé, où chacun est de préférence pur à plus de 97 %.

5. Procédé selon la revendication 4, dans lequel l'étape de séparation comprend en outre les sous-étapes consistant à :
retirer facultativement les solides du mélange de réaction ;
mettre en contact le mélange de réaction avec un solvant organique ;
mettre en contact le solvant avec une base aqueuse diluée ;
séparer le solvant de la base ; et
mettre en contact le solvant avec un milieu chromatographique, de préférence un gel de silice ; et
éluer la théaflavine, le 3-gallate de théaflavine, le 3'-gallate de théaflavine et le 3,3'-digallate de théaflavine, chacun sous forme de composé unique, du milieu chromatographique.

6. Procédé selon la revendication 4 ou 5, dans lequel le rapport polyphénol de thé sur tampon est compris entre 1:5 et 1:20 sur une base kilogramme sur litre, le tampon est un tampon au phosphate à 0,15 M au pH 6,4, et le mélange de réaction est fermenté à une température comprise entre environ 15 °C et environ 80 °C pendant 0,5 à 1,5 heure.

7. Composition pharmaceutique comprenant :
(a) comme agent pharmaceutiquement actif, un mélange isolé de théaflavine, de 3-gallate de théaflavine, de 3'-gallate de théaflavine et de 3,3'-digallate de théaflavine ; et
(b) un ou plusieurs excipients, supports ou diluants physiologiquement acceptables ;
où la composition est destinée à une administration du mélange (a) à une dose dans la gamme de 0,001 mg à 200 mg/kg de poids corporel.

8. Composition pharmaceutique selon la revendication 7, dans laquelle le mélange de théaflavine est présent à un taux compris entre 5 % et 50 % (p/p) dans la composition.

9. Composition pharmaceutique comprenant :
(a) comme agent pharmaceutiquement actif, un mélange de théaflavine, de 3-gallate de théaflavine, de 3'-gallate de théaflavine et de 3,3'-digallate de théaflavine ; et
(b) un ou plusieurs excipients, supports ou diluants physiologiquement acceptables ;
où le mélange de théaflavine est présent à un taux compris entre 5 % et 50 % (p/p) dans la composition.

10. Composition pharmaceutique selon la revendication 9, dans laquelle la composition est destinée à une administration du mélange (a) à une dose dans la gamme de 0,001 mg à 200 mg/kg de poids corporel.

11. Composition pharmaceutique consistant à :
(a) comme agent pharmaceutiquement actif, un mélange de théaflavine, de 3-gallate de théaflavine, de 3'-gallate de théaflavine et de 3,3'-digallate de théaflavine ; et
(b) un ou plusieurs excipients, supports ou diluants physiologiquement acceptables ;
où la composition est destinée à une administration du mélange (a) à une dose dans la gamme de 0,001 mg à 200 mg/kg de poids corporel.

12. Composition pharmaceutique selon la revendication 11, dans laquelle le mélange de théaflavine est présent à un taux compris entre 5 % et 50 % (p/p) dans la composition.

13. Composition pharmaceutique selon l'une quelconque des revendications 7 à 12, dans laquelle la composition est destinée à une administration du mélange (a) à une dose dans la gamme de 0,1 mg à 5 mg/kg de poids corporel.

14. Composition pharmaceutique selon l'une quelconque des revendications 7 à 13, dans laquelle la quantité du mélange (a) par dose est de 70 mg à 210 mg.

15. Composition pharmaceutique selon l'une quelconque des revendications 7 à 14, dans laquelle la composition est destinée à un apport posologique à une seule administration.

16. Composition pharmaceutique selon l'une quelconque des revendications 7 à 15, qui est sous la forme d'une capsule.

17. Composition pharmaceutique selon la revendication 16, qui est sous la forme d'une gélule molle, la composition comprenant le mélange de théaflavine, de la cire d'abeille, de l'huile d'olive et de la gélatine.

18. Composition de complément alimentaire consistant en :
un mélange de théaflavine, de 3-gallate de théaflavine, de 3'-gallate de théaflavine et de 3,3'-digallate de théaflavine ;
et un véhicule de complément alimentaire approprié ;
où la composition est destinée à une administration du mélange (a) à une dose dans la gamme de 0,001 mg à 200 mg/kg de poids corporel.

19. Mélange de théaflavine, de 3-gallate de théaflavine, de 3'-gallate de théaflavine et de 3,3'-digallate de théaflavine pour une utilisation dans un procédé de traitement de prévention d'une hyperlipidémie.

20. Mélange de théaflavine, de 3-gallate de théaflavine, de 3'-gallate de théaflavine et de 3,3'-digallate de théaflavine pour une utilisation dans un procédé de diminution des taux de cholestérol de lipoprotéine de basse densité (LDL-c) sans modifier significativement les taux de cholestérol de lipoprotéine de haute densité (HDL-c).

21. Utilisation d'un mélange de théaflavine, de 3-gallate de théaflavine, de 3'-gallate de théaflavine et de 3,3'-digallate de théaflavine dans la fabrication d'un médicament destiné à traiter ou prévenir une hyperlipidémie.

22. Utilisation d'un mélange de théaflavine, de 3-gallate de théaflavine, de 3'-gallate de théaflavine et de 3,3'-digallate de théaflavine dans la fabrication d'un médicament destiné à diminuer les taux de LDL-c sans modifier significativement les taux de HDL-c.
